# EUROPEAN PATENT APPLICATION

(11) **EP 4 439 003 A1**
(43) Date of publication of application: **02.10.2024**
(21) Application number: 23165436.9
(22) Date of filing: 30.03.2023
(51) Int. Cl.: G01B 9/02015, G01B 9/02056, G01B 9/02091, A61B 3/10

(54) **SWITCHABLE MULTI-CONFIGURATION OCT**

(71) Applicant: Optos PLC, Fife KY11 8GR (GB)
(72) Inventor: BALAGOPAL, Bavishna, Dunfermline, Scotland, KY11 8GR (GB); MUYO, Gonzalo, Dunfermline, Scotland, KY11 8GR (GB)
(74) Representative: Hoffmann Eitle

(57) **Abstract**

An optical coherence tomography, OCT, imaging system for imaging an object, comprising an interferometer having a sample arm, and a reference arm which comprises: a first optical fibre; an optical switch controllable to guide reference light from the first optical fibre to a selected optical path of N optical paths, where N is an integer greater than or equal to 2, each of the N optical paths having a respective optical path length and/or chromatic dispersion that differs from the respective optical path length and/or chromatic dispersion of each of the other optical paths; and an optical coupler to guide the reference light propagating along the selected optical path to a second optical fibre. The OCT imaging system detects an interference between the reference light propagating via the second optical fibre and the sample light propagating via the sample arm after having been scattered by the object.

## Description

### [Field]

Example aspects herein generally relate to the field of optical coherence tomography (OCT) imaging systems.

### [Background]

Optical coherence tomography (OCT) is an imaging technique based on low-coherence interferometry, which is widely used to acquire high-resolution two- and three-dimensional images of optical scattering media, such as biological tissue.

OCT imaging systems can be classified as being time-domain OCT (TD-OCT) or Fourier-domain OCT (FD-OCT) (also referred to as frequency-domain OCT), depending on how depth ranging is achieved. In TD-OCT, an optical path length of a reference arm of the imaging system's interferometer is varied with time during the acquisition of a reflectivity profile of the scattering medium being imaged by the OCT imaging system (referred to herein as the "imaging target"), the reflectivity profile being commonly referred to as a "depth scan" or "axial scan" ("A-scan"). In FD-OCT, a spectral interferogram resulting from an interference between the reference arm and the sample arm of the interferometer at each A-scan location is Fourier transformed to simultaneously acquire all points along the depth of the A-scan, without requiring any variation in the optical path length of the reference arm. FD-OCT can allow much faster imaging than scanning of the sample arm mirror in the interferometer, as all the back reflections from the sample are measured simultaneously. Two common types of FD-OCT are spectral-domain OCT (SD-OCT) and swept-source OCT (SS-OCT). In SD-OCT, a broadband light source delivers many wavelengths to the imaging target, and all wavelengths are measured simultaneously using a spectrometer as the detector. In SS-OCT (also referred to as timeencoded frequency-domain OCT), the light source is swept through a range of wavelengths, and the temporal output of the detector is converted to spectral interference.

OCT imaging systems can also be classified as being point-scan (also known as "point detection" or "scanning point"), line-field or full-field, depending on how the imaging system is configured to acquire OCT data laterally. A point-scan OCT imaging system acquires OCT data by scanning a sample beam, which is focussed to a point on the surface of the imaging target, typically along a single virtual line (which may be straight, or alternatively curved so as to define a circle or a spiral, for example) or along a set of (usually substantially parallel) virtual lines (so-called "scan lines") on the surface of the imaging target, and acquiring an axial depth profile (A-scan) for each of a plurality of points along the line(s), one single point at a time, to build up OCT data comprising a one- or two-dimensional array of A-scans representing a two-dimensional or three-dimensional (volumetric) reflectance profile of the sample. A line-scan OCT imaging system acquires OCT data by generating a line of light, for example by shaping a nominally circular beam of light into a line of light using a cylindrical lens or a Powell lens, for example, scanning the line of light (i.e. providing line-field illumination) across the surface of the imaging target, and acquiring in parallel an axial depth profile (A-scan) for each of a plurality of points along the line, for each scan location of the line, to build up OCT data comprising a one- or two-dimensional array of A-scans representing a two-dimensional or three-dimensional (volumetric) reflectance profile of the sample. A full-field OCT imaging system typically uses a simple halogen lamp (rather than a laser source) and a 2D detector array, such as a CCD camera, to acquire OCT data in the form of tomographic images in the enface (transverse) orientation, which are recorded in parallel by the 2D detector array.

The range of positions, along a direction of propagation of the sample illumination, at which an OCT imaging system can acquire data from an object being imaged, may be determined (at least in part) by the optical path length of the reference arm and the coherence length of the light used to image the imaging target. In ophthalmic applications, OCT imaging systems are usually unable to image both the anterior and posterior segment of the eye without a change being made to the configuration of the interferometric set-up in the OCT imaging system, specifically an adjustment of the reference arm to provide the necessary change in its optical path length. Such changes are usually implemented using mechanical mechanisms which vary the optical path length of the reference arm by moving a mirror provided at an end of the reference arm along an axis of light propagation in the reference arm, for example by use of a linear actuator or the like to move the mirror back and forth along the axis. Such mechanisms are slow to implement the optical path length changes in the reference arm that are often required.

However, some FD-OCT imaging systems have been developed to image the anterior and posterior segments of the eye simultaneously, using full-range OCT techniques that seek to recover the full-imaging depth of the OCT imaging system by resolving the complexconjugate ambiguity induced by the Hermitian Fourier transform of the spectral interferogram. Optical configurations for Dispersion Encoded full-range OCT tend to require a higher level of chromatic dispersion within the reference arm than conventional OCT configurations of the kinds mentioned above, wherein the reference arm can be configured to image either the anterior or posterior segment whilst remaining dispersionmatched with the sample arm.

### [Summary]

There is provided, in accordance with a first example aspect herein, an optical coherence tomography (OCT) imaging system for imaging an imaging target. The OCT imaging system comprises a light source, and an interferometer comprising a sample arm, a reference arm, and an optical splitter arranged to split light from the light source into sample light propagating along the sample arm and reference light propagating along the reference arm. The reference arm comprises: a reference arm optical fibre arranged to guide the reference light; an optical switch controllable to guide at least some of the reference light from the reference arm optical fibre to a selected optical path of N optical paths, where N is an integer greater than or equal to 2, each of the N optical paths having a respective at least one of an optical path length or a chromatic dispersion that differs from the respective at least one of the optical path length or the chromatic dispersion of each of the other optical paths of the N optical paths; and an optical coupler arranged to guide the at least some of the reference light propagating along the selected optical path to an output optical fibre. The OCT imaging system further comprises a photodetector arranged to detect an interference light resulting from an interference between the at least some of the reference light propagating via the output optical fibre and the sample light propagating via the sample arm after having been scattered by the imaging target.

In the OCT imaging system set out above, the optical switch may be a 1 × N optical switch which comprises N output ports, the optical coupler may be a N × 1 optical coupler which comprises N input ports, and each of the N optical paths may comprise a respective optical fibre connecting a respective one of the N output ports to a respective one of the N input ports. At least some of the optical fibres may have different optical path lengths. Additionally or alternatively, each of one or more of the N optical paths may comprise a respective dispersive element, wherein each dispersive element of the dispersive elements is arranged to provide a respective level of chromatic dispersion of the reference light propagating through the optical path that comprises the dispersive element. The N × 1 optical coupler may comprise an N × 1 optical fibre coupler or an (active) N × 1 optical switch which is controllable to couple, to the output optical fibre, an input port of the N input ports corresponding to the output port of the N output ports to which the reference light has been coupled by the 1 × N optical switch.

The OCT imaging system or any of its variants set out above may further comprise a controller, and the OCT imaging system may be operable in multiple imaging modes to image different respective ranges of depths of the imaging target along a propagation direction of the sample light towards the imaging target. In this case, during operation of the OCT imaging system in each of the imaging modes, the controller may be arranged to control the optical switch to guide the reference light from the reference arm optical fibre to a respective one of the N optical paths which has a respective optical path length such that differences in phase of the sample light that is received at the photodetector after having been scattered from depths within a respective range of depths of the imaging target, and the reference light that is received at the photodetector after having propagated via the output optical fibre, is less than a predetermined threshold. Furthermore, the imaging target may be an eye, and the OCT imaging system may be operable in at least one of: a first imaging mode to image a first portion of an anterior segment of the eye; a second imaging mode to image a first portion of a posterior segment of the eye; or a third imaging mode to image a second portion of the anterior segment of the eye and a second portion of the posterior segment of the eye.

Where the OCT imaging system is operable in the first imaging mode, the controller may be arranged to control the optical switch to guide the reference light from the reference arm optical fibre to a first optical path of the N optical paths, which comprises a first dispersive element configured such that a level of chromatic dispersion of the sample light that is received at the photodetector after having been scattered by the first portion of the anterior segment of the eye, matches a level of chromatic dispersion of the reference light (L_{R}) that is received at the photodetector after having propagated via the output optical fibre.

Where the OCT imaging system is operable in the second imaging mode, the controller may be arranged to control the optical switch to guide the reference light from the reference arm optical fibre to a second optical path of the N optical paths, which comprises a second dispersive element configured such that a level of chromatic dispersion of the sample light that is received at the photodetector after having been scattered by the first portion of the posterior segment of the eye, matches a level of chromatic dispersion of the reference light that is received at the photodetector after having propagated via the output optical fibre.

Where the OCT imaging system is operable in the third imaging mode, the controller may be arranged to control the optical switch to guide the reference light from the reference arm optical fibre to a third optical path of the N optical paths, which provides a level of chromatic dispersion of the reference light that is received at the photodetector after having propagated via the output optical fibre, which is greater than a level of chromatic dispersion of the sample light that is received at the photodetector after having been scattered by the second portion of the anterior segment of the eye and/or the second portion of the posterior segment.

The OCT imaging system may further comprise a lens and a lens movement mechanism for moving the lens into and out of an optical path in the sample arm of the interferometer. In the first imaging mode, the controller may be arranged to control the lens movement mechanism to move the lens into the optical path in the sample arm of the interferometer, to allow the first portion of the anterior segment of the eye to be imaged via the lens during operation of the OCT imaging system in the first imaging mode. In the second imaging mode, the controller may be arranged to control the lens movement mechanism to move the lens out of the optical path in the sample arm of the interferometer, to allow the first portion of the posterior segment of the eye to be imaged without use of the lens during operation of the OCT imaging system in the second imaging mode. Alternatively, the OCT imaging system may further comprise a pupil alignment module arranged to bring a focal point of the OCT imaging system into alignment with a pupil of the eye based on images of the anterior ocular segment. In this case, the OCT imaging system may be arranged to operate in the first imaging mode during operation of the pupil alignment module to bring the focal point of the OCT imaging system into alignment with the pupil of the eye, and in the second imaging mode after the operation of the pupil alignment module to bring the focal point of the OCT imaging system into alignment with the pupil of the eye.

In the OCT imaging system or any of its variants set out above, the optical switch may comprise a micro-electromechanical switch, a micro-mechanical switch, a micro-optical switch and/or an optical switch driven by a stepper motor. Additionally or alternatively, the OCT imaging system may further comprise an optical power monitor, and the optical switch may be controllable to: (i) simultaneously guide a first portion of the reference light from the reference arm optical fibre to the selected optical path of the N optical paths, and a second portion of the reference light from the reference arm optical fibre to the optical power monitor, and/or (ii) switch between guiding the at least a portion of the reference light from the reference arm optical fibre to the selected optical path of the N optical paths, and guiding the at least a portion of the reference light (L_{R}) from the reference arm optical fibre to the optical power monitor.

There is also provided, in accordance with a second example aspect herein, an OCT imaging system for imaging an imaging target. The OCT imaging system comprises a light source, and an interferometer comprising a sample arm, a reference arm, and an optical splitter arranged to split light from the light source into sample light propagating along the sample arm and reference light propagating along the reference arm. The reference arm comprises: a first mirror arranged to reflect reference light from the optical splitter back towards the optical splitter when the reference light from the optical splitter is incident on the first mirror; a second mirror; and a mirror movement mechanism controllable to move the second mirror into and out of an optical path of the reference light propagating towards the first mirror, such that the reference light is reflected back towards the optical splitter by the first mirror when the second mirror has been moved out of the optical path, and such that the reference light is reflected back towards the optical splitter by the second mirror instead of the first mirror when the second mirror has been moved into the optical path. The OCT imaging system further comprises a photodetector arranged to detect an interference light resulting from an interference between the reference light reflected back towards the optical splitter and the sample light propagating via the sample arm after having been scattered by the imaging target.

The OCT imaging system of the second example aspect may be operable in a first imaging mode to image a first range of depths of the imaging target along a propagation direction of the sample light towards the imaging target, and in a second imaging mode to image a second range of depths of the imaging target along the propagation direction of the sample light towards the imaging target, the first range of depths being different from the second range of depths. The OCT imaging system may further comprise a controller arranged to control the mirror movement mechanism such that: during operation of the OCT imaging system in the first imaging mode, the second mirror is out of the optical path such that a difference in phase of the sample light that is received at the photodetector after having been scattered from the first range of depths of the imaging target, and the reference light that is received at the photodetector after having been reflected back towards the optical splitter by the first mirror, is less that a predetermined threshold; and during operation of the OCT imaging system in the second imaging mode, the second mirror is in the optical path such that a difference in phase of the sample light that is received at the photodetector after having been scattered from the second range of depths of the imaging target, and the reference light that is received at the photodetector after having been reflected back towards the optical splitter by the second mirror, is less that the predetermined threshold. The OCT imaging system may be operable in the first imaging mode to image, as the first range of depths of the imaging target, a portion of a posterior segment of an eye, and in the second imaging mode to image, as the second range of depths of the imaging target, a portion of an anterior segment of the eye.

There is also provided, in accordance with a third example aspect herein, a computer-implemented method of controlling imaging of an imaging target by an OCT imaging system, the OCT imaging system comprising: a light source, and an interferometer comprising a sample arm, a reference arm, and an optical splitter arranged to split light from the light source into sample light propagating along the sample arm and reference light propagating along the reference arm. The reference arm comprises: a reference arm optical fibre arranged to guide the reference light; an optical switch controllable to guide at least some of the reference light from the reference arm optical fibre to a selected optical path of N optical paths, where N is an integer greater than or equal to 2, each of the N optical paths having a respective at least one of an optical path length and a chromatic dispersion that differs from the at least one of the optical path length and the chromatic dispersion of the other optical paths of the N optical paths; and an optical coupler arranged to guide the at least some of the reference light propagating along the selected optical path to an output optical fibre. The OCT imaging system further comprises a photodetector arranged to detect an interference light resulting from an interference between the at least some of the reference light output from the output optical fibre and the sample light propagating via the sample arm after having been scattered by the imaging target. The computer-implemented method comprises: receiving a signal indicative of a range of imaging depths across which an image of a portion of the imaging target is to be acquired; configuring the OCT imaging system to acquire the image of the portion of the imaging target across the indicated range of imaging depths, by: selecting an optical path of the N optical paths, based on the received signal; controlling the optical switch to guide the reference light from the reference arm optical fibre to the selected optical path. The computer-implemented method further comprises controlling the configured OCT imaging system to acquire the image of the portion of the imaging target.

There is also provided a computer program comprising computer-readable instructions which, when executed by a computer, causes the computer to perform the method set out above. The computer program may be stored in a non-transitory computer-readable storage medium or carried by a signal.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an OCT imaging system according to a first example embodiment herein.
Figure 2 is a schematic illustration of a scanning system forming part of the OCT imaging system of the first example embodiment.
Figure 3 is a schematic illustration of an example implementation of the reference arm in the example embodiment.
Figure 4 is a schematic illustration of an example implementation of the reference arm in an alternative example embodiment.
Figure 5 is a schematic illustration of programmable signal processing hardware, which may be configured to perform at least some of the functions of the OCT data processing hardware described herein.
Figure 6 is a flow diagram illustrating a process by which a controller of the OCT imaging system of the first example embodiment controls the OCT imaging system to image an imaging target.
Figure 7 is a schematic illustration of an OCT imaging system according to a second example embodiment herein.

### [Detailed Description of Example Embodiments]

Example embodiments of an OCT imaging system described herein address at least some shortcomings of conventional OCT imaging systems that have been recognised by the present inventors, including an inability to image an imaging target over a wide range of depths (e.g. imaging over substantially the full depth range of the human eye, from the anterior segment to the posterior segment) or to switch between a full-range imaging mode and one or more standard OCT imaging modes (having narrower depth-of-field and a requirement for dispersion matching between the sample and reference arms of the interferometer), without requiring time-consuming changes to be made to the configuration of the interferometric set-up in the OCT imaging system as discussed above, which can make the OCT imaging system less efficient to use.

More specifically, the present inventors have devised an OCT imaging system wherein light in the reference arm of the interferometer of the OCT imaging system can be switched to propagate along a selected optical path of multiple different optical paths that are available in the reference arm. Each of the available optical paths may have a respective optical path length and/or level of chromatic dispersion set to meet the requirements of an associated imaging mode of the OCT imaging system. The imaging mode of the OCT imaging system may be changed simply by controlling an optical switch to switch between the available optical paths in the reference arm so as to provide the required optical path length and/or level of chromatic dispersion. For example, in ophthalmic applications, the proposed OCT imaging system may switch between OCT imaging system configurations for operating in an imaging mode for imaging the anterior segment of the eye, another imaging mode for imaging the posterior segment of the eye and a further imaging mode for full-range OCT imaging of the eye.

Example embodiments of an OCT imaging system and a method of controlling imaging of an imaging target by an OCT imaging system will now be described in detail, with reference to accompanying drawings.

### [First Example Embodiment]

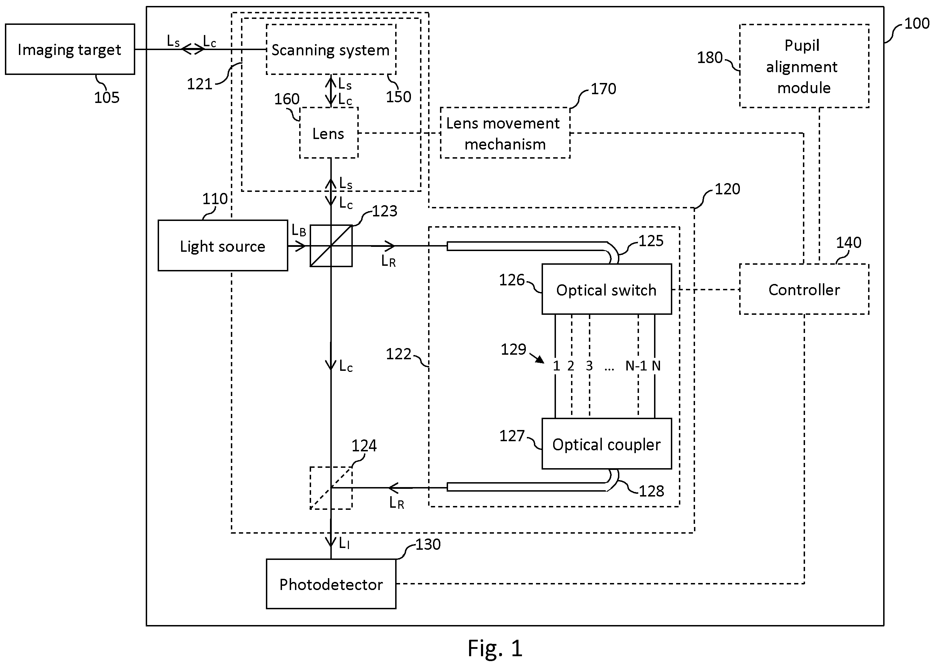

Figure 1 is a schematic illustration of an OCT imaging system 100 for imaging an imaging target (object) 105 which may, as in the present example embodiment, be a human eye. The OCT imaging system 100 comprises a light source 110, an interferometer 120 and a photodetector 130. The OCT imaging system 100 may, as in the present example embodiment, further comprise a controller 140. Furthermore, the OCT imaging system 100 may, as in the present example embodiment, be a point-scan swept-source OCT (SS-OCT) imaging system, which further comprises a scanning system 150. However, the OCT imaging system 100 may, more generally, be any OCT imaging system known in the art, such as any Fourier-domain OCT (FD-OCT) imaging system, as described below, which may be a point-scan, line-scan or full-field OCT imaging system that images a portion of an eye (or other object) using a mirror-based optical system, a lens-based optical system or an optical system comprising at least one mirror and at least one lens.

The light source 110 may, as in the present example embodiment, be a swept light source in the form of a wavelength-swept (or "tuneable") laser, for example, which is arranged to generate a beam of light L_{B} having a wavelength that is swept over a range of wavelengths (preferably linearly) during imaging of the imaging target 105 by the OCT imaging system 100. The tuneable laser may be of type known to those versed in the art, such as one based on a Fourier-domain mode-locking (FDML) laser with a Fabry-Perot tuneable filter or polygonal scanning mirror, or a microcavity tuneable laser with microelectromechanical systems (MEMS), for example. The median frequency of the swept source light source is selected in dependence on the imaging target 105 and is usually in the near-infrared or infrared part of the spectrum (typically about 1050 nm) in ophthalmic applications, for example. Alternatively, in example embodiments where the OCT imaging system 100 takes the form of an SD-OCT imaging system, the light source 110 may be a broadband light source in the form of a super-luminescent diode, for example, which is arranged to generate a beam of light L_{B} simultaneously having a range of wavelengths (i.e. a broad spectral content) during imaging of the imaging target 105 by the OCT imaging system 100. In some example embodiments, the light source 110 may comprise further components, such as one or more cylindrical lenses to shape the beam of light L_{B}, a light source aperture and/or one or more collimating lenses for collimating the beam of light L_{B}, for example.

The interferometer 120 comprises a sample arm 121, a reference arm 122, a first optical splitter 123 and may, as in the present example embodiment, comprise a second optical splitter 124. The first optical splitter 123 is arranged to split light L_{B} from the light source 110 into sample light Ls propagating along the sample arm 121 and reference light L_{R} propagating along the reference arm 122. The sample light Ls propagating along the sample arm 121 in a first direction is scattered by the imaging target 105. The sample light propagating via the sample arm 121 after having been scattered by the imaging target 105 (i.e. the collected light L_{C}, which is at least some of the light scattered by the imaging target 105 during imaging of the imaging target 105) propagates along the sample arm 121 in a second direction, opposite the first direction, towards the first optical splitter 123. The sample light L_{S} may, as in the present example embodiment, be guided to the imaging target 105 by the scanning system 150 and the collected light Lc may be collected by the scanning system 150. The collected light Lc passes through the first optical splitter 123 towards the second optical splitter 124. The second optical splitter 124 is arranged to combine the collected light Lc and the reference light L_{R} to form an inference light L_{I} resulting from an interference between the reference light L_{R} exiting the reference arm 122 and the collected light Lc. The interference light L_{I} propagates towards the photodetector 130.

The first optical splitter 123 and the second optical splitter 124 may, as in the present example embodiment, comprise beam splitters, as shown. However, the first optical splitter 123 and the second optical splitter 124 are not so limited, and may instead take the form of a 2 × 2 fibre coupler (e.g. a 50:50 fibre coupler) or a 2 × 1 fibre coupler, respectively. Further, in some example embodiments, the second optical splitter 124 may be omitted, for example in a case where the reference arm 122 guides the reference light L_{R} to a mirror, which is arranged to reflect the reference light L_{R} back through the reference arm 122 to the first optical splitter 123, the first optical splitter 123 being arranged to combine the collected light Lc and the reference light L_{R} to form an inference light L_{I} resulting from an interference between the reference light L_{R} exiting the reference arm 122 and the collected light Lc.

The scanning system 150 may, as in the present example embodiment, be arranged to perform a two-dimensional point-scan of the beam of light Ls across the imaging target 105 and collect light L_{C} which has been scattered by the imaging target 105 during the point scan. The scanning system 150 is therefore arranged to acquire A-scans at respective scan locations that are distributed two-dimensionally across a surface of the imaging target 105, by sequentially illuminating the scan locations with the beam of light L_{S}, one scan location at a time, and collecting at least some of the light L_{C} scattered by the imaging target 105 at each scan location. The scanning system 150 may perform the two-dimensional point-scan using any suitable scan pattern known to those versed in the art, for example a unidirectional scan (wherein a set of parallel scan lines are followed in a common direction, along which they extend), a serpentine scan or spiral scan.

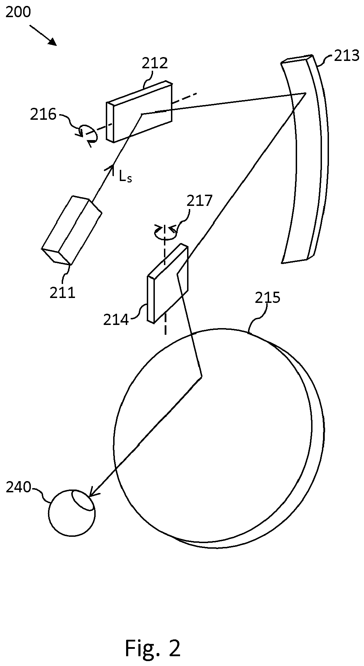

The scanning system 150 may, as in the present example embodiment, be a mirror-based system which comprises a scanning element and a mirror, wherein the scanning system 150 is arranged to perform the two-dimensional point scan by the scanning element scanning the beam of light Ls across the imaging target 105 via the mirror. An example of such a scanning system, which is capable of performing a wide-field retinal scan, is described in WO 2014/53824 A1, the content of which is hereby incorporated by reference in its entirety. The scanning system 150 may, as in the present example embodiment, be provided in the form of scanning system 200 shown in Figure 2. The scanning system 200 comprises an optical coupler 211, a first scanning element 212, a first curved mirror 213, a second scanning element 214 and a second curved mirror 215. The beam of light L_{S} enters the scanning system 200 via the optical coupler 211. The beam of light L_{S} is then reflected, in sequence, by the first scanning element 212, the first curved mirror 213, the second scanning element 214 and the second curved mirror 215, before being incident on the imaging target that is in the form of an eye 220. The light Lc which has been scattered by the eye 220 and collected by the scanning system 200 follows the same optical path through the scanning system 200 as the beam of light Ls but in reverse order, and exits the scanning system 200 via the optical coupler 211. It is noted that the scanning system 150 may alternatively be a lens-based system, wherein the scanning system 150 is arranged to perform the two-dimensional point scan by the one or more scanning elements scanning the beam of light Ls across the imaging target 105 via one or more lenses. Such lens-based scanning systems are well-known to those versed in the art, and are therefore not described in further detail herein.

The two-dimensional point scan is performed by the first scanning element 212 rotating around a first axis 216 to scan the beam of light Ls in a first direction across the imaging target, and by the second scanning element 214 rotating around a second axis 217 to scan the beam of light L_{S} in a second direction across the imaging target (which may, as in the present example embodiment, be orthogonal to the first direction). Thus, by rotating the first scanning element 212 and the second scanning element 214, it is possible to steer the beam of light L_{S} to any position on the imaging target. The rotation of the first scanning element 212 and the second scanning element 214 may be coordinated by a scanning system controller, which may be the controller 140 or a separate controller (not shown), such that the beam of light L_{S} is scanned across the imaging target in accordance with a predefined scan pattern, as discussed above.

The first curved mirror 213 is an ellipsoidal mirror (and referred to as a slit mirror), and the second curved mirror 215 is also an ellipsoidal mirror. Each of the ellipsoidal mirrors has two focal points. The first scanning element 212 is disposed at a first focal point of the first curved mirror 213, and the second scanning element 214 is disposed at a second focal point of the first curved mirror 213. The second scanning element 214 is also disposed at a first focal point of the second curved mirror 215, and the eye 220 (more specifically, the pupil of the eye 220 in the present example) is disposed at a second focal point of the second curved mirror 215. However, either curved mirror may instead be any reflective component having an aspherical reflective surface, such as a shape of a conical section like a parabola or hyperboloid, or may, more generally, have a shape described by one or more polynomial functions of two variables.

The first scanning element 212 and the second scanning element 214 may, as in the present example embodiment, each be a galvanometer optical scanner (or "galvo"), although another type of scanning element could alternatively be used, such as a MEMS scanning mirror or a resonant scanning mirror, for example.

Referring again to Figure 1, the reference arm 122 comprises a reference arm optical fibre 125, an optical switch 126 and an optical coupler 127. The reference arm optical fibre 125 is arranged to guide the reference light L_{R} (from the optical splitter 121 to the optical switch 126). The reference arm 122 may, as in the present example embodiment, further comprise an output optical fibre 128 arranged to guide the reference light L_{R} towards the photodetector 130 (via the second optical splitter 124).

The optical switch 126 is controllable (for example by controller 140, as in the present example embodiment) to guide the reference light L_{R} from the reference arm optical fibre 125 to a selected optical path of N optical paths 129, where N is an integer greater than or equal to 2. Each of the N optical paths 129 has a respective optical path length and/or chromatic dispersion that differs from the respective optical path length and/or chromatic dispersion of each of the other optical paths of the N optical paths. In other words, each of the N optical paths either has an optical path length which differs from the optical path lengths of each of the other optical paths of the N optical paths, a chromatic dispersion which differs from the chromatic dispersions of each of the other optical paths of the N optical paths, or a combination of an optical path length and a chromatic dispersion which differs from the combinations of the respective optical path lengths and chromatic dispersions of each of the other optical paths of the N optical paths. The optical switch 126 may, as in the present example embodiment, be controllable by the controller 140, although it may instead be controllable by hand (i.e. by the user of the OCT imaging system 100 operating a mechanical switch, or dial, which is operable to change the switch setting of the optical switch 126). The optical coupler 127 is arranged to guide the reference light L_{R} propagating along the selected optical path to the output optical fibre 128.

The optical switch 126 may, as in the present example embodiment, be a 1 × N optical switch (i.e. a 1 × N optical fibre switch) having N output ports that are optically coupled to respective ones of the N optical paths described above, although it may alternatively be a 1 × M optical switch (where M is an integer greater than N) that includes these N output ports as well as one or more additional output ports. For example, in some example embodiments, where M = N + 1, the 1 × M optical switch has one additional output port which is connected to an optical power monitor arranged to monitor an optical power of the reference light L_{R} propagating through the reference arm optical fibre 125 when the 1 × M optical switch is set to direct the light from the reference arm optical fibre 125 to the aforementioned additional output port. In other example embodiments, where M = N + P, the 1 × M optical switch has P additional output ports (where P is an integer greater than or equal to 2), where each of the P additional output ports is connected to a respective one of P optical power monitors, each optical power monitor being arranged to monitor an optical power of light incident thereon when the 1 × M optical switch is set to direct the light from the reference arm optical fibre 125 to the aforementioned additional P output ports. The provision of multiple optical power monitors provides redundancy (in case one of the optical power monitors fails or performs incorrectly), allowing the optical power of the reference light L_{R} propagating through the reference arm optical fibre 125 to be monitored with greater reliability.

The optical switch 126 may be controllable to simultaneously guide a first portion of the reference light L_{R} from the reference arm optical fibre 125 to the selected optical path of the N optical paths 129 (so that only some of the reference light L_{R} from the reference arm optical fibre 125 is channelled to the selected optical path), and a second portion of the reference light L_{R} from the reference arm optical fibre 125 (which second portion may be the remainder of the reference light L_{R}) to the optical power monitor 390, or a respective portion of the reference light L_{R} from the reference arm optical fibre 125 to the respective optical power monitor, in case more than one optical power monitor 390 is provided, as noted above. Additionally or alternatively, optical switch 126 may be controllable to switch between guiding the reference light L_{R} (or a portion thereof) from the reference arm optical fibre 125 to the selected optical path of the N optical paths 129, and guiding the reference light L_{R} (or a portion thereof) from the reference arm optical fibre 125 to the optical power monitor(s) 390.

The optical power monitor(s) may be any kind of photoelectric device that can generate an electric signal indicative of the optical power of a received optical signal, and may include a Si, Ge or InGaAs detector, for example. Furthermore, the form of optical switch 126 is not limited, and the optical switch 126 may comprise a micro-electromechanical switch, a micro-mechanical switch, a micro-optical switch and/or an optical switch driven by a stepper motor, for example.

The optical coupler 127 may, as in the present example embodiment, be a N × 1 optical coupler having N input ports that are optically coupled to respective ones of the N optical paths described above, although it may alternatively be a M × 1 optical coupler (where M is an integer greater than N) that has the N input ports as well as one or more additional input ports. For example, the N × 1 optical coupler may either be an N × 1 optical fibre coupler or an N × 1 optical switch (controllable by the controller 140 or by hand, as described above) to couple, to the output optical fibre 128, an input port of the N input ports corresponding to the output port of the N output ports to which the reference light L_{R} has been coupled by the optical switch 126. The form of the optical coupler 127 is not limited, and the optical coupler 127 may comprise a micro-electromechanical switch, a micro-mechanical switch, a micro-optical switch, an optical switch driven by a stepper motor or a passive T-coupler, for example.

Each of the N optical paths 129 may, as in the present example embodiment, comprise a respective optical fibre connecting a respective one of the N output ports to a respective one of the N input ports. At least some of the optical fibres may, as in the present example embodiment, have different optical path lengths (by having different respective physical lengths and/or be made of materials have different respective refractive indices).

Accordingly, by this arrangement, the optical path of the reference light L_{R} propagating along the reference arm 122 may be altered by the selection of an appropriate optical fibre connecting one of the N output ports of the optical switch 126 to a corresponding one of the N input ports of the optical coupler 127. However, this alteration of optical path length in the reference arm 122 may also be achieved using free-space optics in some or all of the N optical paths 129, for example by use of mirrors (not shown) to increase the distance of light propagating along an optical path between the selected output port of the optical switch 126 and the corresponding input port of the optical coupler 127.

Each of one or more of the N optical paths 129 may, as in the present example embodiment, comprise a respective dispersive element. Each dispersive element of the dispersive elements is arranged to provide a respective level of chromatic dispersion of the reference light L_{R} propagating through the optical path that comprises the dispersive element. For example, each dispersive element may be a glass rod or an optical fibre that provides the required level of chromatic dispersion, and may be placed between two portions (e.g. halves) of the respective optical fibre connecting a respective output port of the optical switch 126 to the corresponding input port of the optical coupler 127. Accordingly, each of the one or more optical paths may have their respective level of chromatic dispersion set by the inclusion of a respective dispersive element that provides the required level of chromatic distortion. However, instead of using dispersive elements, a level of chromatic dispersion of the reference light L_{R} propagating through an optical path of the N optical paths 129 may instead be provided by an optical fibre connecting the corresponding output port of the optical switch 126 to the respective input port of the optical coupler 127 (e.g. by the selection of an optical fibre which has a predetermined level of chromatic dispersion, such as by choice of a specific type of fibre given the length of the fibre necessary to achieve the desired optical path length).

Furthermore, in some example embodiments, each of one or more of the N optical paths 129 may comprise a respective dispersive element without including an optical fibre, the dispersion element thus being arranged to provide a respective level of chromatic dispersion of the reference light L_{R} propagating through the optical path that comprises the dispersive element. In such a case, the alteration to the optical path length of the optical path may instead be achieved by the dispersive element itself, as described above, and/or by free-space optics.

By an appropriate selection of optical fibres and/or dispersive elements in at least some of the N optical paths 129, as described above, each optical path of the N optical paths may thus have a respective optical path length and/or chromatic dispersion that differs from the respective optical path length and/or chromatic dispersion of each of the other optical paths of the N optical paths 129. Namely, the optical path length of an optical path of the N optical paths 129 may be set to a predetermined value by appropriately setting the optical path length of the optical fibre in the optical path whilst taking into account the optical path lengths of any other elements in the optical path (such as a dispersive element, where provided), and the level of chromatic dispersion of that optical path may be set to a predetermined level by appropriately setting the level of chromatic dispersion of a selected dispersive element in the optical path whilst taking into account the respective levels of chromatic dispersion of other elements in the optical path (including the level of chromatic dispersion of an optical fibre connecting a respective one of the N output ports to a respective one of the N input ports, where provided for the optical path). Thus, the predetermined value of the optical path length and/or level of chromatic dispersion of the optical path can be set to differ from each of the other optical paths of the N optical paths 129.

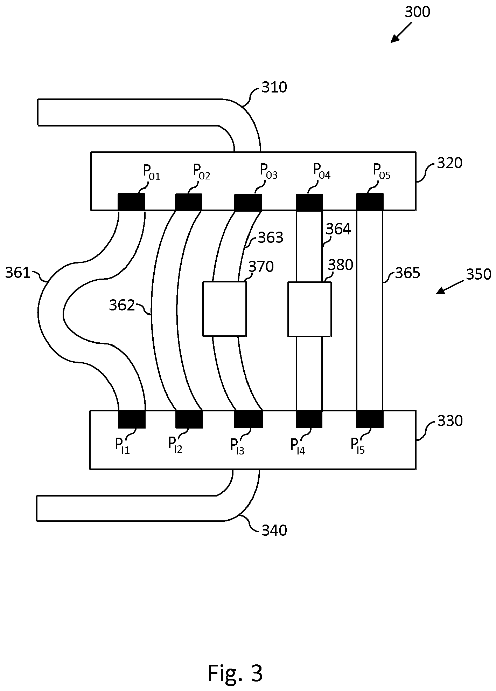

The reference arm 122 may be provided in the form of reference arm 300 that is illustrated schematically in Figure 3. The reference arm 300 comprises a reference arm optical fibre 310, a 1 × 5 optical switch 320, a 5 × 1 optical coupler 330, an output optical fibre 340 and five optical paths 350. The 1 × 5 optical switch 320 comprises five output ports P_{O1}, P_{O2}, P_{O3}, P_{O4}, and P_{O5}, and the 5 × 1 optical coupler 330 comprises five input ports P_{I1}, P_{I2}, P_{I3}, P_{I4}, and P_{I5}. Each optical path of the optical paths 350 is provided between a respective output port of the 1 × 5 optical switch 320 and a respective likenumbered input port of the 5 × 1 optical coupler 330 (e.g. a first optical path is provided between ports P_{O1} and P_{I1}). The optical paths 350 comprise respective optical fibre 361, 362, 363, 364 and 365 (of the same type) joining each respective output port to its corresponding input port. The third and fourth optical paths from the left in Figure 3 comprise dispersive elements 370 and 380, respectively, which are provided between two halves of the optical fibres 363 and 364 that are provided in the third and fourth optical paths. The dispersive elements 370 and 380 provide a different pre-selected level of chromatic dispersion. Further, as shown in Figure 3, optical fibre 361 is of a first length, optical fibres 362 and 363 are of a different second length, and optical fibres 364 and 365 are of a third length that is different from the first and second length. Accordingly, each of the five optical paths 350 has a respective unique combination of optical path length and chromatic dispersion. Although the example reference arm 300 of Figure 3 is provided with five optical paths, fewer or more optical paths could alternatively be provided, each of which has a respective optical path length and/or chromatic dispersion that differs from optical path length and/or chromatic dispersion of each of the other optical paths.

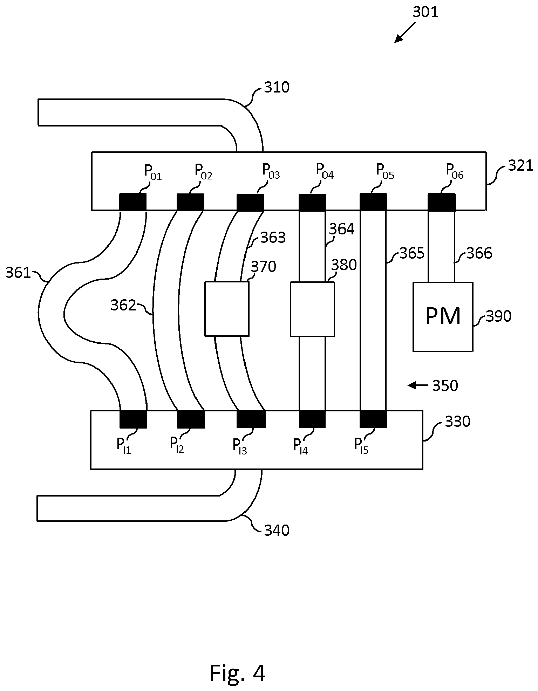

The reference arm 122 may alternatively be provided in the form of reference arm 301 that is illustrated schematically in Figure 4. The reference arm 301 of Figure 4 differs from reference arm 300 of Figure 3 only by having a 1 × 6 optical switch 321 in place of the 1 × 5 optical switch 320, where the additional output port P₀₆ of the 1 × 6 optical switch 321 is optically coupled to a power monitor 390 via an optical fibre 366. The power monitor 390 may take any of the forms described above (among others). The power monitor 390 may be used to monitor the optical power of the reference light L_{R} propagating through the reference arm optical fibre 125 when the 1 × 6 optical switch 321 is set to direct the light from the reference arm optical fibre 125 to the additional output port P₀₆, for example for the purpose of checking that the measured power level corresponds to a safe level of illumination being delivered to a patient's eye by the light source 110. The controller 140 may be arranged to power off the light source 110 or otherwise stop illumination of the patient's eye by the light source 110 (by closing a shutter, for example) in case the optical power measured by the power monitor 390 exceeds a predetermined threshold level.

By the above-described arrangement of the reference arm 122 to be switchable between N optical paths 129 having varying optical path lengths and/or levels of chromatic dispersion may be advantageous in enabling the OCT imaging system 100 to switch between different optical configurations to allow the OCT imaging system 100 to operate in multiple imaging modes, which would previously have required dedicated OCT imaging systems or time-consuming configuration changes within a single OCT imaging system for each configuration. The OCT imaging system 100 may, as in the present example embodiment, be operable in multiple imaging modes to image different respective ranges of depths of the imaging target 105 along a propagation direction of the sample light Ls towards the imaging target 105. For example, where the imaging target 105 is an eye, each range of depths may correspond to a respective layer of the eye whose thickness direction is along the optical axis of the eye.

During operation of the OCT imaging system 100 in each of the imaging modes, the controller 140 may be arranged to control the optical switch 126 to guide the reference light L_{R} from the reference arm optical fibre 125 to a respective one of the N optical paths which has a respective optical path length such that an absolute value of difference between a phase of the sample light Lc received at the photodetector 130 after having been scattered from depths within a respective range of depths of the imaging target 105, and a phase of the reference light L_{R} received at the photodetector 130 after having propagated via the output optical fibre 128, is less than a predetermined threshold. In other words, the respective one of the N optical paths has an optical path length such that (the absolute value of) the optical path length difference between the optical path length of the sample arm 121 (including the optical path to and within the imaging target 105) and the optical path length of the reference arm 122, is less than the predetermined threshold, for each respective range of depths of the imaging target 105 that is imaged in each imaging mode. The predetermined threshold may, for example, be a predetermined fraction of the coherence length of the light generated by the light source 110 (i.e. the beam of light L_{B}) or may be the coherence length of the light generated by the light source 110 itself. Accordingly, the OCT imaging system 100 may be operable to image various different ranges of depths of the imaging target 105 by switching between the N optical paths, to bring the optical path difference between the arms of the interferometer 120 within the coherence length of the light generated by the light source 110.

Further, by the use of dispersive elements within one or more of the optical paths, the OCT imaging system 100 may be configured to operate in multiple imaging modes, wherein, in one or more of the imaging modes, the level of chromatic dispersion between the arms of the interferometer 120 is mis-matched, for example in full-range OCT imaging mode. High dispersion in the reference arm of the interferometer 120 serves to modify the phase of the reference beam and make it match the phase of backscattered light from the imaging target 105. The high dispersion can alternatively be incorporated into sample arm 121. For example, in Dispersion Encoded full-range OCT (DE-FR-OCT), the phase of the signal is encoded by the dispersion element in the imaging system. The algorithm used in DE-FR-OCT assumes an initial phase and, based on a recorded intensity and phase of the detected OCT signal, updates the estimated phase iteratively. When the estimated phase converges, the original signal is restored (based on pre-determined criteria), while the mirror image degrades and can be removed to retrieve depth information. In one or more other imaging modes for imaging more limited depth ranges of the imaging target 105, the chromatic dispersion between the arms of the interferometer 120 may be matched.

The imaging target 105 may, as in the present example embodiment, be a human eye, and the OCT imaging system 100 may be operable in a first imaging mode to image at least a portion of the anterior segment of the eye, a second imaging mode to image at least a portion of the posterior segment of the eye, and/or a third imaging mode to (simultaneously) image at least a portion of the anterior segment of the eye and at least a portion of the posterior segment of the eye. However, whilst the OCT imaging system 100 is described herein as an ophthalmic OCT imaging system, the imaging target 105 is not so limited and may, alternatively, be any tissue (e.g. skin), biological sample or, more generally, any scattering medium whose sub-subsurface structure is to be imaged by OCT. In such cases, the imaging modes may correspond to portions of the imaging target at different ranges of depths, or to the use of full-range OCT techniques to image across an increased imaging depth.

Where the OCT imaging system 100 is operable in the first imaging mode, the controller 140 may, as in the present example embodiment, be arranged to control the optical switch 126 to guide the reference light L_{R} from the reference arm optical fibre 125 to a first optical path of the N optical paths, which comprises a first dispersive element configured such that a level of chromatic dispersion of the sample light Lc received at the photodetector 130 after having been scattered by the anterior segment of the eye, matches a level of chromatic dispersion of the reference light L_{R} received at the photodetector 130 has after having propagated via the output optical fibre 128. Accordingly, in the first imaging mode, the optical path selected by the controller 140 results in an optical path difference between the sample arm 121 and the reference arm 122 which is less than the coherence length of the light generated by the light source 110 for the respective range of depths imaged in the anterior segment of the eye, and the level of chromatic dispersion in the sample arm 121 (not factoring in any chromatic dispersion induced by the eye) is matched to that in the reference arm 122. By matching the chromatic dispersion between the arms of the interferometer 120 in the first imaging mode, the OCT imaging system 100 may image the anterior segment of the eye (or a portion thereof) without full-range OCT techniques, which may reduce the computational expense and improve imaging quality, as image artefacts can be better controlled due to the autocorrelation from the OCT imaging system 100. In this regard, algorithms used in full-range OCT tend to degrade the SNR as they can introduce artefacts that need to be suppressed. In the present example embodiment, providing chromatic dispersion matching between the arms of the interferometer 120 may allow the posterior segment of the eye to be imaged, and/or segments of the anterior segment to be imaged sequentially, without the use of the aforementioned algorithms.

The OCT imaging system 100 may, as in the present example embodiment, further comprise a lens 160 for changing focus settings for imaging the anterior and posterior segments of the eye, for example, and a lens movement mechanism 170 for moving the lens 160 into and out of an optical path in the sample arm 121 of the interferometer 120. In such a case, the controller 140 may be arranged to control the lens movement mechanism 170 to move the lens 160 into the optical path in the sample arm 121 of the interferometer 120, to allow the anterior segment of the eye (or a portion thereof) to be imaged via the lens 160 during operation of the OCT imaging system 100 in the first imaging mode. Further, the lens movement mechanism 170 may be arranged to adjust the position of the lens 160 to vary the focal point of the lens 160. In such cases, the controller 140 may control the lens movement mechanism 170 to optimise the focus of the OCT imaging system 100 when operating in the first imaging mode. The provision of the lens 160 aids in allowing the anterior segment of the eye to be imaged by the OCT imaging system 100 in the first imaging mode.

Where the OCT imaging system 100 is operable in the second imaging mode, the controller 140 may, as in the present example embodiment, be further arranged to control the optical switch 126 to guide the reference light L_{R} from the reference arm optical fibre 125 to a second optical path of the N optical paths, which comprises a second dispersive element configured such that a level of chromatic dispersion of the sample light L_{C} received at the photodetector 130 after having been scattered by the posterior segment of the eye (including a portion of the retina, for example), matches a level of chromatic dispersion of the reference light L_{R} received at the photodetector 130 after having propagated via the output optical fibre 128. Accordingly, in the second imaging mode, the optical path selected by the controller 140 results in an optical path difference between the sample arm 121 and the reference arm 122 which is less than the coherence length of the light generated by the light source 110 for the respective range of depths in the posterior segment of the eye that has been imaged, and the level of chromatic dispersion in the sample arm 121 (not factoring any chromatic dispersion induced by the eye) is matched to that in the reference arm 122. By matching the chromatic dispersion between the arms of the interferometer 120 in the second imaging mode, the OCT imaging system 100 is configured to image the posterior segment of the eye (or a portion thereof) without full-range OCT techniques, which may reduce the computational expense and improve imaging quality, as image artefacts can be better controlled due to the autocorrelation coming from the OCT imaging system 100, as described above.

The controller 140 may, as in the present example embodiment, be further arranged to control the lens movement mechanism 170 to move the lens 160 out of the optical path in the sample arm 121 of the interferometer 120, to allow the posterior segment of the eye (or portion thereof) to be imaged without use of the lens 160 during operation of the OCT imaging system 100 in the second imaging mode. The OCT imaging system 100 can thus perform imaging using the lens 160 in the first imaging mode, and without using the lens 160 in the second imaging mode. Accordingly, the anterior segment of the eye may be imaged in the first imaging mode by the OCT imaging system 100 using the lens 160, without impeding on the ability of the OCT imaging system 100 to image the posterior segment of the eye, where use of the lens 160 is not required.

Where the OCT imaging system 100 is operable in the third imaging mode, the controller 140 may, as in the present example embodiment, be further arranged to control the optical switch 126 to guide the reference light L_{R} from the reference arm optical fibre 125 to a third optical path of the N optical paths, which provides a level of chromatic dispersion of the reference light L_{R} received at the photodetector 130 after having propagated via the output optical fibre 128, that is greater than the level of chromatic dispersion of the sample light L_{C} received at the photodetector 130 after having been scattered by the anterior segment of the eye and/or the posterior segment of the eye. Accordingly, in the third imaging mode, the optical path selected by the controller 140 results in an optical path difference between the sample arm 121 and the reference arm 122 which is less than the coherence length of the light generated by the light source 110 for the respective range of depths imaged across the anterior segment of the eye (or portion thereof) and the posterior segment of the eye (or portion thereof), and the level of chromatic dispersion in the reference arm 122 is greater than that in the sample arm 121 (not factoring in any chromatic dispersion induced by the imaging target 105). By mis-matching the chromatic dispersion between the arms of the interferometer 120 in the third imaging mode, the OCT imaging system 100 may image the anterior and posterior segments of the eye (or portions thereof) with full-range OCT techniques, as described above.

While three imaging modes have been described above, the OCT imaging system 100 is not so limited and may, more generally, use the switchable functionality of the OCT imaging system 100 to switch between various optical paths arranged to provide the optical path length and/or level of chromatic dispersion required for any particular set of system requirements, whether as dependent upon the imaging depth, the OCT technique used or otherwise. For example, the OCT imaging system 100 may additionally or alternatively comprise a fourth imaging mode which is the same as the first imaging mode other than including a fourth dispersive element (instead of the first dispersive element) arranged to provide a level of chromatic dispersion in the reference arm 122 as described in the third imaging mode, a fifth imaging mode which is the same as the second imaging mode other than including a fifth dispersive element (instead of the second dispersive element) arranged to provide a level of chromatic dispersion in the reference arm 122 as described in the third imaging mode or a sixth imaging mode which is the same as the third imaging mode other than including a sixth dispersive element (instead of the third dispersive element) arranged to provide a level of chromatic dispersion in the reference arm 122 as described in the first imaging mode or second imaging mode. Accordingly, in the fourth and fifth imaging mode, full-range OCT techniques can be used in the same manner as with the third imaging mode, which may be desirable for capturing an increased imaging depth within the respective segment of the eye. Further, in the sixth imaging mode, at least a part of the anterior segment of the eye and the posterior segment of the eye may be imaged without full-range OCT techniques, for example where the range of depths to be imaged across the anterior segment of the eye and the posterior segment of the eye is achievable without the need for the increased imaging depth range obtained with full-range OCT techniques.

Referring again to Figure 1, the photodetector 130 is arranged to detect an interference light L_{I} resulting from an interference between the reference light L_{R} propagating via the output optical fibre 128 and the sample light L_{C} propagating via the sample arm 121 after having been scattered by the imaging target 105. In other words, the reference light L_{R} and the collected light L_{C} are guided to interfere with one another at the optical splitter 122 during imaging of the imaging target 105, and the resulting interference light L_{I} is directed to and received by the photodetector 130. The photodetector 130 is arranged to generate a detection signal based on the measured interference, which may be processed by the controller 140, or separate OCT data processing hardware, to generate images of the imaging target 105 using well-known OCT data processing techniques. For example, the controller 140 may generate complex volumetric OCT data and use this to produce enface images of the imaging target 105, by well-known projection techniques such as summed-voxel projection (SVP), or restricted SVP (RSVP), which restricts the SVP to a selected slab of the imaged sample.

The photodetector 130 generates the detection signal by performing photoelectric conversion of the measured interference between the reference light L_{R} and the sample light Lc propagating via the sample arm 121 after having been scattered by the imaging target 105. The specific form which the photodetector 130 may take will depend on the form in which the OCT imaging system 100 is implemented. For example, where the OCT imaging system 100 is implemented as an SD-OCT imaging system, the photodetector 130 may comprise a spectrometer, which may have a diffraction grating, Fourier transform lens, and a detector array (or a line scan camera). Where the OCT imaging system 100 is implemented as a SS-OCT imaging system, as in the present example embodiment, the photodetector 130 may comprise a balanced photodetector set-up comprising two photodetectors (e.g. reverse-biased photodiodes), whose output photocurrents are subtracted from one another, with the subtracted current signal being converted into a voltage detection signal by a transimpedance amplifier.

The OCT imaging system 100 may, as in the present example embodiment, further comprise a pupil alignment module 180, which is arranged to bring a focal point of the OCT imaging system 100 into alignment with a pupil of the eye based on images of the anterior ocular segment, using well-known techniques. The OCT imaging system 100 may, as in the present example embodiment, be arranged to operate in the first imaging mode during operation of the pupil alignment module 180 to bring the focal point of the OCT imaging system 100 into alignment with the pupil of the eye, and in the second imaging mode (or the third imaging mode) after the operation of the pupil alignment module 180 to bring the focal point of the OCT imaging system 100 into alignment with the pupil of the eye. Thus, after the pupil alignment module 180 has brought the focal point of the OCT imaging system 100 into alignment with the pupil of the eye, the controller 140 is arranged to control the optical switch 126 to switch, from guiding the reference light L_{R} from the reference arm optical fibre 125 to the first optical path, to guiding the reference light L_{R} from the reference arm optical fibre 125 to the second optical path.

The ability of the OCT imaging system 100 to switch between different imaging modes may allow the OCT imaging system to advantageously use images from the anterior segment of the eye for patient alignment before quickly switching to the second imaging mode (or the third imaging mode) described above to acquire the OCT images of interest, which may improve the OCT imaging workflow significantly. The workflow time for patient alignment may be further reduced by not using the lens 160 while imaging in the first imaging mode during the patient alignment, as the images acquired without use of the lens 160 may be sufficient for patient alignment purposes.

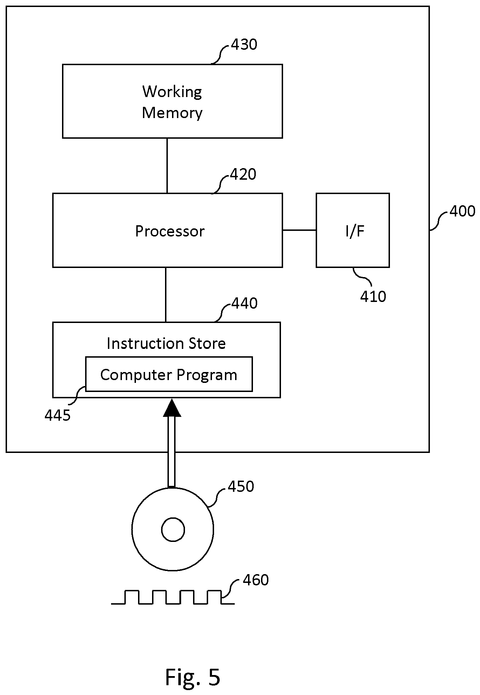

The controller 140 may be provided in any suitable form, for example as a programmable signal processing hardware 400 of the kind illustrated schematically in Figure 5. The programmable signal processing apparatus 400 comprises a communication interface (I/F) 410, for use in receiving instructions as to the imaging mode in with the OCT imaging system 100 should operate and in outputting a signal to the optical switch 126 (and, optionally, the N × 1 optical switch as the optical coupler 127), or for use in receiving the detection signal from the photodetector 130 and outputting the images of the imaging target 105 (for example, in the form of an enface projection) for displaying on a display, such a computer screen or the like. The signal processing hardware 400 further comprises a processor (e.g., a Central Processing Unit, CPU, and/or a Graphics Processing Unit, GPU) 420, a working memory 430 (e.g. a random-access memory) and an instruction store 440 storing a computer program 445 comprising the computer-readable instructions which, when executed by the processor 420, cause the processor 420 to perform various functions including those of the controller 140 described herein. The working memory 430 stores information used by the processor 420 during execution of the computer program 445. The instruction store 440 may comprise a ROM (e.g., in the form of an electrically erasable programmable read-only memory (EEPROM) or flash memory) which is pre-loaded with the computer-readable instructions. Alternatively, the instruction store 440 may comprise a RAM or similar type of memory, and the computer-readable instructions of the computer program 445 can be input thereto from a computer program product, such as a non-transitory, computer-readable storage medium 450 in the form of a CD-ROM, DVDROM, etc. or a computer-readable signal 460 carrying the computer-readable instructions. In any case, the computer program 445, when executed by the processor 420, causes the processor 420 to perform the functions of the controller 140 as described herein. In other words, the controller 140 of the example embodiment may comprise a computer processor 420 and a memory 440 storing computer-readable instructions which, when executed by the computer processor 420, cause the computer processor 420 to control the optical switch 126 to guide the reference light L_{R} from the reference arm optical fibre 125 to a selected optical path of N optical paths, optionally controlling the N × 1 optical switch simultaneously with the optical switch 126, as described above. Further, the computer-readable instructions, when executed by the computer processor 420, may cause the computer processor 420 to process the detection signal to generate images of the imaging target 105.

It should be noted, however, that the controller 140 may alternatively be implemented in non-programmable hardware, such as an ASIC, an FPGA or other integrated circuit dedicated to performing the functions of the controller 140 described above, or a combination of such non-programmable hardware and programmable hardware as described above with reference to Figure 5.

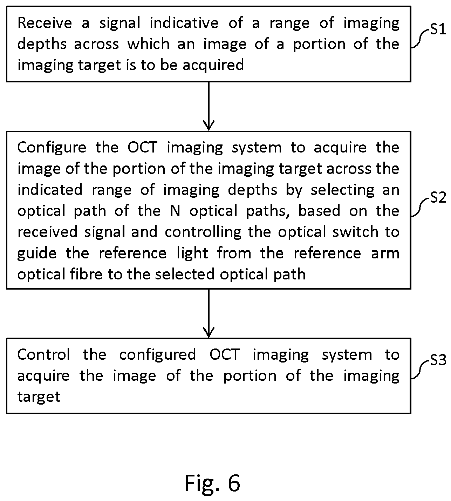

Figure 6 is a flow diagram illustrating a process by which the controller 140 of the present example embodiment controls imaging of the imaging target 105 by the OCT imaging system 100.

In step S1 of Figure 6, the controller 140 receives a signal indicative of a range of imaging depths across which an image of a portion of the imaging target 105 is to be acquired. The received signal may, as in the present example embodiment, be indicative of the range of imaging depths corresponding to either of the first imaging mode, the second imaging mode or the third imaging mode, as described above.

In step S2 in Figure 6, the controller 140 configures the OCT imaging system 100 to acquire the image of the portion of the imaging target 105 across the indicated range of imaging depths. To do this, the controller 140 selects an optical path of the N optical paths, based on the received signal, and controls the optical switch 126 to guide the reference light L_{R} from the reference arm optical fibre 125 to the selected optical path. Where the optical coupler 127 is provided in the form of an N × 1 optical switch, as described above, the controller 140 may further N × 1 optical switch to switch simultaneously with the optical switch 126 to connect a selected output port of the optical switch 126 to a corresponding input port of the N × 1 optical switch. For example, when the received signal is indicative of the range of imaging depths corresponding to the first imaging mode, the controller 140 select the optical path corresponding to the optical path length and/or chromatic dispersion required for the first imaging mode.

In step S3 of Figure 6, the controller 140 controls the configured OCT imaging system 100 to acquire the image of the portion of the imaging target 105. This is achieved by operating the configured OCT imaging system 100 using techniques well-known to those versed in the art. For example, the controller 140 may, as in the present example embodiment, control the configured OCT imaging system 100 to acquire the image of the portion of the imaging target 105 by operating the pupil alignment module 180, operating the movement module 170, controlling the scanning system 105 to perform the two-dimensional point scan of the imaging target 105 across the indicated range of imaging depths, receiving the detection signal from the photodetector 130, and generating the image of the portion of the imaging target 105, based on the detection signal, as described above.

The process of Figure 6 may be performed by controller 140 implemented in the form of a programmable signal processing hardware 400 as described above with reference to Figure 5, which operates in accordance with instructions included in the computer program 445 when the computer program 445 is executed by the processor 420. This computer program 445 may be stored in a computer program product 450, such as a non-transitory, computer-readable storage medium in the form of a CD-ROM, DVDROM, etc. or a computer-readable signal 460 carrying the computer-readable instructions.

### [Second Example Embodiment]

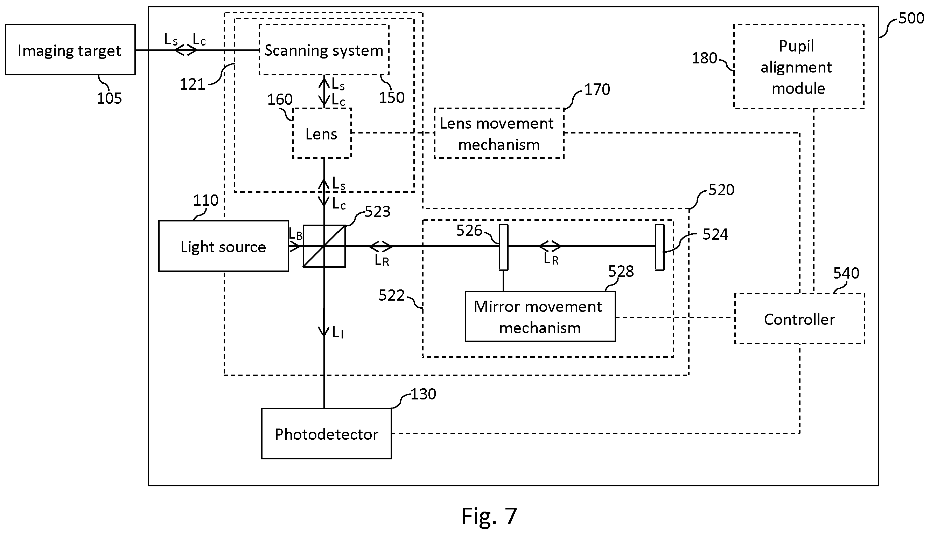

Figure 7 is schematic illustration of an OCT imaging system 500 for imaging an imaging target 105 according to a second example embodiment. The OCT imaging system 500 comprises a light source 110, an interferometer 520, a controller 540 and a photodetector 130 and may, as in the present example embodiment, further comprise a lens 160, a lens movement mechanism 170, and a pupil alignment module 180. The OCT imaging system 500 may, as in the present example embodiment, be a point-scan SS-OCT imaging system which further comprises a scanning system 150. However, the OCT imaging system 500 may, more generally, be any OCT imaging system known in the art, such as any FD-OCT imaging system, for example.

The light source 110, a sample arm 121 of interferometer 520, the photodetector 130, the lens 160, the lens movement mechanism 170 and the pupil alignment module 180 are the same as in the first example embodiment. These components, and other components of the OCT imaging system 500 that are labelled with the same reference numerals as in the first example embodiment of Figure 1, will therefore not be described again. The present example embodiment differs from the first example embodiment of Figure 1 by the configuration, at least in part, of parts of the interferometer 520 (in particular, in the reference arm 522 of the interferometer 520), and the differences are described below with reference to Figure 7. Further, the controller 540 differs from the controller 140 only by its control of the reference arm 522.

The interferometer 520 differs from the interferometer 120 of the first example embodiment only by the configuration of the reference arm 522 and in comprising an optical splitter 523 in place of both the optical splitter 123 and the optical splitter 124 of the first example embodiment. The optical splitter 523 is arranged to split light L_{B} from the light source 110 into sample light L_{S} propagating along the sample arm 121 and reference light L_{R} propagating along the reference arm 522, and to combine the collected light L_{C} from the sample arm 121 and the reference light L_{R} exiting the reference arm 522 to form an inference light L_{I} resulting from an interference between the reference light L_{R} and the collected light L_{C}.

The reference arm 522 comprises a first mirror 524, a second mirror 526 and a mirror movement mechanism 528. The first mirror 524 is arranged to reflect reference light L_{R} from the optical splitter 523 back towards the optical splitter 523 when the reference light L_{R} from the optical splitter 523 is incident on the first mirror 524.

The mirror movement mechanism 528 is controllable by the controller 540 to move the second mirror 526 into and out of an optical path of the reference light L_{R} propagating towards the first mirror 524, such that the reference light L_{R} is reflected back towards the optical splitter 523 by the first mirror 524 when the second mirror 526 has been moved out of the optical path, and such that the reference light L_{R} is reflected back towards the optical splitter 523 by the second mirror 526 instead of the first mirror 524 when the second mirror 526 has been moved into the optical path. In other words, the controller 540 controls the mirror movement mechanism 528 to move the second mirror 526 to and from a position which is in front of the first mirror 524 and within the optical path of the reference light L_{R} propagating towards the first mirror 524 such that the second mirror 526, instead of the first mirror 524, reflects the reference light L_{R} back towards the optical splitter 523 when the second mirror 526 is at that position. The mirror movement mechanism 528 may, as in the present example embodiment, rotate the second mirror 526 into and out of an optical path of the reference light L_{R} propagating towards the first mirror 524 (e.g. about an axis of rotation which is parallel to the optical path, so as to reduce or eliminate movement of the second mirror 526 in a direction along the optical path), although the mirror movement mechanism 528 may alternatively translate the second mirror 526 into and out of the aforementioned optical path (e.g. in a direction that is perpendicular to the optical path, so as to reduce or eliminate movement of the second mirror 526 in a direction along the optical path). By either of these arrangements, the optical path length of the reference arm 522 may be switched between two values by the controller 540, which may be selected as required for a particular OCT imaging system configuration. As compared to conventional mechanical mechanisms of the kind described above, which vary the optical path length of a reference arm by moving a mirror provided at an end of the reference arm along the optical path of the reference light by use of a linear actuator or the like, the present arrangement may allow large optical path length changes to be made relatively quickly, by reducing the distance that a mirror in the reference arm is required to travel to effect the required optical path length change.

The OCT imaging system 500 may, as in the present example embodiment, be operable in a first imaging mode to image a first range of depths of the imaging target 105 along a propagation direction of the sample light L_{S} towards the imaging target 105, and in a second imaging mode to image a second range of depths of the imaging target 105 along the propagation direction of the sample light L_{S} towards the imaging target 105. The first range of depths is different from the second range of depths, but the two ranges may partially overlap in some cases. For example, in the present example embodiment, wherein the imaging target 105 is an eye, the ranges of depths are ranges of depths along the optical axis of the eye. Further, in such a case, the OCT imaging system 500 may be operable in the first imaging mode to image, as the first range of depths of the imaging target 105, a portion of the anterior segment of an eye and in the second imaging mode to image, as the second range of depths of the imaging target 105, a portion of the posterior segment of the eye.

The controller 540 may, as in the present example embodiment, be arranged to control the mirror movement mechanism 528 such that, during operation of the OCT imaging system 500 in the first imaging mode, the second mirror 526 is out of the optical path such that a difference in phase of the sample light L_{C} received at the photodetector 130 after having been scattered from the first range of depths of the imaging target 105, and the reference light L_{R} received at the photodetector has after having been reflected back towards the optical splitter 523 by the first mirror 524, is less that a predetermined threshold. In other words, the reference arm 522, when the second mirror 526 is moved out of the optical path of the reference light L_{R}, has an optical path length such that the optical path length difference between the optical path length of the sample arm 121 and the optical path length of the reference arm 522 is less than a predetermined threshold across the first range of depths of the imaging target 105 that are imaged in the first imaging mode.

The controller 540 may, as in the present example embodiment, be further arranged to control the mirror movement mechanism 528 such that, during operation of the OCT imaging system 500 in the second imaging mode, the second mirror 526 is in the optical path such that a difference in phase of the sample light L_{C} received at the photodetector 130 has after having been scattered from the second range of depths of the imaging target, and the reference light L_{R} received at the photodetector after having been reflected back towards the optical splitter 523 by the second mirror 526, is less that the predetermined threshold. In other words, the reference arm 522, when the second mirror 526 is moved into of the optical path of the reference light L_{R}, has an optical path length such that the optical path length difference between the optical path length of the sample arm 121 and the optical path length of the reference arm 522 is less than a predetermined threshold across the second range of depths of the imaging target 105 that are imaged in the second imaging mode.

The predetermined threshold may, for example, be a predetermined fraction of the coherence length of the light generated by the light source 110 or may be the coherence length of the light generated by the light source 110 itself. Accordingly, the OCT imaging system 100 may switch between imaging two ranges of depths of the imaging target 105 by switching the second mirror 526 into and out of the optical path of the reference light L_{R} propagating towards the first mirror 524. This switch brings the optical path difference between the arms of the interferometer 120 below the coherence length of the light as appropriate for the respective range of depths.

While the second example embodiment has been described with two mirrors in the reference arm 522, the OCT imaging system 500 is not so limited and may employ additional mirrors, movable by the mirror movement mechanism 528 (or by a separate movement mechanism controllable by the controller 540 in a similar manner), to produce other, differing optical path lengths in the reference arm 522. Accordingly, the OCT system 500 may quickly switch between operation in a variety of imaging modes that allow imaging of the imaging target 105 across various respective ranges of depths.

While the first example embodiment and the second example embodiment have been described with reference to point-scan SS-OCT systems, the OCT imaging systems are not so limited. For example, either of the described OCT imaging systems may be a point-scan, line-scan or full-field OCT system. In the case of a line-scan system, the light source 110 would be replaced by a line-source arranged to generate a line of light, and the scanning system 150 would be arranged to perform scan of the line of light across the imaging target 105. In the case of the full-field system, the light source 110 would generates a beam of light and the scanning system 150 could be replaced with a transfer optical system arranged to guide the beam of light to the imaging target 105 and collect scattered light during imaging of the imaging target 105. Further, any of the OCT imaging systems described above may be provided in the alternative form of a SD-OCT system, by using appropriate arrangements of the light source 110 and the photodetector 130, as described above. Further, any of the OCT imaging systems described above may be provided in the alternative form of a TD-OCT imaging system. For example, in the first example embodiment, the second optical splitter 124 may be omitted in favour of a mirror, as described above, and the second mirror may be made (translationally) movable by a further movement mechanism (controllable by the controller 140) to acquire image data with the OCT imaging system 100 at differing respective depths within the imaging target 105 in the manner of the TD-OCT imaging systems well-known to those versed in the art. The first mirror 524 and the second movable mirror 526 may be similarly (translationally) movable where the OCT imaging system 500 of the second example embodiment is adapted to form a TD-OCT imaging system.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the function of the controller, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the controller may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims and their equivalents.

Further, the purpose of the Abstract is to enable the Patent Office and the public generally, and especially the scientists, engineers and practitioners in the art who are not familiar with patent or legal terms or phraseology, to determine quickly from a cursory inspection the nature and essence of the technical disclosure of the application. The Abstract is not intended to be limiting as to the scope of the example embodiments presented herein in any way. It is also to be understood that any procedures recited in the claims need not be performed in the order presented.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. An optical coherence tomography, OCT, imaging system (100) for imaging an imaging target (105), the OCT imaging system (100) comprising:
a light source (110);
an interferometer (120) comprising a sample arm (121), a reference arm (122), and an optical splitter (123) arranged to split light from the light source (110) into sample light (Ls) propagating along the sample arm (121) and reference light (L_{R}) propagating along the reference arm (122),
wherein the reference arm (122) comprises:
a reference arm optical fibre (125) arranged to guide the reference light (LR);
an optical switch (126) controllable to guide at least some of the reference light (L_{R}) from the reference arm optical fibre (125) to a selected optical path of N optical paths (129), where N is an integer greater than or equal to 2, each of the N optical paths (129) having a respective at least one of an optical path length or a chromatic dispersion that differs from the respective at least one of the optical path length or the chromatic dispersion of each of the other optical paths of the N optical paths (129); and
an optical coupler (127) arranged to guide the at least some of the reference light (L_{R}) propagating along the selected optical path to an output optical fibre (128); and
a photodetector (130) arranged to detect an interference light (L_{I}) resulting from an interference between the at least some of the reference light (L_{R}) propagating via the output optical fibre (128) and the sample light (Lc) propagating via the sample arm (121) after having been scattered by the imaging target (105).

2. The OCT imaging system (100) according to Claim 1, wherein the optical switch (126) is a 1 × N optical switch which comprises N output ports, the optical coupler (127) is a N × 1 optical coupler which comprises N input ports, and each of the N optical paths (129) comprises a respective optical fibre connecting a respective one of the N output ports to a respective one of the N input ports.

3. The OCT imaging system (100) according to Claim 2, wherein at least some of the optical fibres have different optical path lengths.

4. The OCT imaging system (100) according to any preceding claim, wherein each of one or more of the N optical paths (129) comprises a respective dispersive element, wherein each dispersive element of the dispersive elements is arranged to provide a respective level of chromatic dispersion of the reference light (L_{R}) propagating through the optical path that comprises the dispersive element.

5. The OCT imaging system (100) according to any of Claims 2 to 4, wherein the N × 1 optical coupler comprises one of:
an N × 1 optical fibre coupler; and
an N × 1 optical switch controllable to couple, to the output optical fibre (128), an input port of the N input ports corresponding to the output port of the N output ports to which the reference light (L_{R}) has been coupled by the 1 × N optical switch.

6. The OCT imaging system (100) according to any preceding claim, further comprising a controller (140), wherein
the OCT imaging system (100) is operable in multiple imaging modes to image different respective ranges of depths of the imaging target (105) along a propagation direction of the sample light (Ls) towards the imaging target (105), and
during operation of the OCT imaging system (100) in each of the imaging modes, the controller (140) is arranged to control the optical switch (126) to guide the reference light (L_{R}) from the reference arm optical fibre (125) to a respective one of the N optical paths (129) which has a respective optical path length such that differences in phase of the sample light (Lc) that is received at the photodetector (130) after having been scattered from depths within a respective range of depths of the imaging target (105), and the reference light (L_{R}) that is received at the photodetector (130) after having propagated via the output optical fibre (128), is less than a predetermined threshold.

7. The OCT imaging system (100) according to Claim 6, wherein imaging target (105) is an eye, and the OCT imaging system (100) is operable in at least one of:
a first imaging mode to image a first portion of an anterior segment of the eye;
a second imaging mode to image a first portion of a posterior segment of the eye; or
a third imaging mode to image a second portion of the anterior segment of the eye and a second portion of the posterior segment of the eye.

8. The OCT imaging system (100) according to Claim 7, wherein
where the OCT imaging system (100) is operable in the first imaging mode, the controller (140) is arranged to control the optical switch (126) to guide the reference light (L_{R}) from the reference arm optical fibre (125) to a first optical path of the N optical paths (129), which comprises a first dispersive element configured such that a level of chromatic dispersion of the sample light (Lc) that is received at the photodetector (130) after having been scattered by the first portion of the anterior segment of the eye, matches a level of chromatic dispersion of the reference light (L_{R}) that is received at the photodetector (130) after having propagated via the output optical fibre (128),
where the OCT imaging system (100) is operable in the second imaging mode, the controller (140) is arranged to control the optical switch (126) to guide the reference light (L_{R}) from the reference arm optical fibre (125) to a second optical path of the N optical paths (129), which comprises a second dispersive element configured such that a level of chromatic dispersion of the sample light (Lc) that is received at the photodetector (130) after having been scattered by the first portion of the posterior segment of the eye, matches a level of chromatic dispersion of the reference light (L_{R}) that is received at the photodetector (130) after having propagated via the output optical fibre (128), and
where the OCT imaging system (100) is operable in the third imaging mode, the controller (140) is arranged to control the optical switch (126) to guide the reference light (L_{R}) from the reference arm optical fibre (125) to a third optical path of the N optical paths (129), which provides a level of chromatic dispersion of the reference light (L_{R}) that is received at the photodetector (130) after having propagated via the output optical fibre (128), which is greater than a level of chromatic dispersion of the sample light (Lc) that is received at the photodetector (130) after having been scattered by the second portion of the anterior segment of the eye and/or the second portion of the posterior segment.

9. The OCT imaging system (100) according to Claim 7 or 8, further comprising:
a lens (160) and a lens movement mechanism (170) for moving the lens (160) into and out of an optical path in the sample arm (121) of the interferometer (120), wherein
in the first imaging mode, the controller (140) is arranged to control the lens movement mechanism (170) to move the lens (160) into the optical path in the sample arm (121) of the interferometer (120), to allow the first portion of the anterior segment of the eye to be imaged via the lens (160) during operation of the OCT imaging system (100) in the first imaging mode; and
in the second imaging mode, the controller (140) is further arranged to control the lens movement mechanism (170) to move the lens (160) out of the optical path in the sample arm (121) of the interferometer (120), to allow the first portion of the posterior segment of the eye to be imaged without use of the lens (160) during operation of the OCT imaging system (100) in the second imaging mode.

10. The OCT imaging system (100) according to Claim 8, further comprising:
a pupil alignment module (180) arranged to bring a focal point of the OCT imaging system (100) into alignment with a pupil of the eye based on images of the anterior ocular segment, wherein
the OCT imaging system (100) is arranged to operate in the first imaging mode during operation of the pupil alignment module (180) to bring the focal point of the OCT imaging system (100) into alignment with the pupil of the eye, and in the second imaging mode after the operation of the pupil alignment module (180) to bring the focal point of the OCT imaging system (100) into alignment with the pupil of the eye.

11. The OCT imaging system (100) according to any preceding claim, further comprising an optical power monitor (390), wherein the optical switch (126) is controllable to:
simultaneously guide a first portion of the reference light (L_{R}) from the reference arm optical fibre (125) to the selected optical path of the N optical paths (129), and a second portion of the reference light (L_{R}) from the reference arm optical fibre (125) to the optical power monitor (390), and/or
switch between guiding the at least a portion of the reference light (L_{R}) from the reference arm optical fibre (125) to the selected optical path of the N optical paths (129), and guiding the at least a portion of the reference light (L_{R}) from the reference arm optical fibre (125) to the optical power monitor (390).

12. An optical coherence tomography, OCT, imaging system (500) for imaging an imaging target (105), the OCT imaging system (500) comprising:
a light source (110);
an interferometer (520) comprising a sample arm (121), a reference arm (522), and an optical splitter (123) arranged to split light from the light source (110) into sample light (Ls) propagating along the sample arm (121) and reference light (L_{R}) propagating along the reference arm (522),
wherein the reference arm (522) comprises:
a first mirror (524) arranged to reflect reference light (L_{R}) from the optical splitter (523) back towards the optical splitter (523) when the reference light (L_{R}) from the optical splitter (523) is incident on the first mirror (524);
a second mirror (526); and
a mirror movement mechanism (528) controllable to move the second mirror (526) into and out of an optical path of the reference light (L_{R}) propagating towards the first mirror, such that the reference light (L_{R}) is reflected back towards the optical splitter (523) by the first mirror (524) when the second mirror (526) has been moved out of the optical path, and such that the reference light (L_{R}) is reflected back towards the optical splitter (523) by the second mirror (526) instead of the first mirror (524) when the second mirror (526) has been moved into the optical path; and
a photodetector (130) arranged to detect an interference light (L_{I}) resulting from an interference between the reference light (L_{R}) reflected back towards the optical splitter (523) and the sample light (Lc) propagating via the sample arm (121) after having been scattered by the imaging target (105).

13. The OCT imaging system (500) according to Claim 12, wherein
the OCT imaging system (500) is operable in a first imaging mode to image a first range of depths of the imaging target (105) along a propagation direction of the sample light (Ls) towards the imaging target (105), and in a second imaging mode to image a second range of depths of the imaging target (105) along the propagation direction of the sample light (Ls) towards the imaging target (105), the first range of depths being different from the second range of depths,
the OCT imaging system (500) further comprises a controller (540) arranged to control the mirror movement mechanism (528) such that
during operation of the OCT imaging system (500) in the first imaging mode, the second mirror (526) is out of the optical path such that a difference in phase of the sample light (Lc) that is received at the photodetector (130) after having been scattered from the first range of depths of the imaging target (105), and the reference light (L_{R}) that is received at the photodetector (130) after having been reflected back towards the optical splitter (523) by the first mirror (524), is less that a predetermined threshold, and
during operation of the OCT imaging system (500) in the second imaging mode, the second mirror (526) is in the optical path such that a difference in phase of the sample light (Lc) that is received at the photodetector (130) after having been scattered from the second range of depths of the imaging target (105), and the reference light (L_{R}) that is received at the photodetector (130) after having been reflected back towards the optical splitter (523) by the second mirror (526), is less that the predetermined threshold.

14. The OCT imaging system (500) according to Claim 13, wherein the OCT imaging system (500) is operable in the first imaging mode to image, as the first range of depths of the imaging target (105), a portion of a posterior segment of an eye, and in the second imaging mode to image, as the second range of depths of the imaging target (105), a portion of an anterior segment of the eye.

15. A computer-implemented method of controlling imaging of an imaging target (105) by an optical coherence tomography, OCT, imaging system (100), the OCT imaging system (100) comprising:
a light source (110);
an interferometer (120) comprising a sample arm (121), a reference arm (122), and an optical splitter (123) arranged to split light from the light source (110) into sample light (Ls) propagating along the sample arm (121) and reference light (L_{R}) propagating along the reference arm (122);
wherein the reference arm (122) comprises:
a reference arm optical fibre (125) arranged to guide the reference light (LR);
an optical switch (126) controllable to guide at least some of the reference light (L_{R}) from the reference arm optical fibre (143) to a selected optical path of N optical paths (129), where N is an integer greater than or equal to 2, each of the N optical paths (129) having a respective at least one of an optical path length and a chromatic dispersion that differs from the at least one of the optical path length and the chromatic dispersion of the other optical paths of the N optical paths (129); and
an optical coupler (127) arranged to guide the at least some of the reference light (L_{R}) propagating along the selected optical path to an output optical fibre (128); and
a photodetector (130) arranged to detect an interference light (L_{I}) resulting from an interference between the at least some of the reference light (L_{R}) output from the output optical fibre (128) and the sample light (Lc) propagating via the sample arm (121) after having been scattered by the imaging target (105),
the method comprising:
receiving (51) a signal indicative of a range of imaging depths across which an image of a portion of the imaging target (105) is to be acquired;
configuring (S2) the OCT imaging system (100) to acquire the image of the portion of the imaging target (105) across the indicated range of imaging depths, by:
selecting an optical path of the N optical paths (129), based on the received signal; and
controlling the optical switch (126) to guide the reference light (L_{R}) from the reference arm optical fibre (125) to the selected optical path; and
controlling (S3) the configured OCT imaging system (100) to acquire the image of the portion of the imaging target (105).
